# EUROPEAN PATENT APPLICATION

(11) **EP 3 777 680 A1**
(43) Date of publication of application: **17.02.2021**
(21) Application number: 19192138.6
(22) Date of filing: 16.08.2019
(51) Int. Cl.: A61B 5/145, A61B 5/1455, A61B 5/00

(54) **ACTIVE MINIATURIZED SENSING SYSTEM AND METHOD**

(71) Applicant: Glucomat GmbH, 93309 Kelheim (DE)
(72) Inventor: Reichl, Mathias, 93326 Abensberg (DE)
(74) Representative: Weickmann & Weickmann PartmbB

(57) **Abstract**

The present invention relates to a non-invasive active sensing system for determining a physiological parameter in a bodily fluid of subject. Further, the present invention relates to a non-invasive method for determining a physiological parameter in a bodily fluid of a subject.

## Description

The present invention relates to a non-invasive active sensing system for determining a physiological parameter in a bodily fluid of subject. Further, the present invention relates to a non-invasive method for determining a physiological parameter in a bodily fluid of a subject.

### Background

In the year 2016, about 415 million people suffered from diabetes. For 2040, an increase to more than 640 million people can be expected. Since people with diabetes are at risk for complications such as blindness, kidney diseases, heart diseases and stroke, there is a need to control the disease by closely monitoring blood glucose level.

Presently, determination of blood glucose is mainly based on invasive system and methods, wherein either a blood sample is taken and subsequently subjected to an *in vitro* test or a sensor is implanted for determining the glucose level *in vivo.* These invasive systems and methods are disadvantageous in that they are painful or inconvenient.

Thus, there is a need for developing a system and methods which allow a reliable non-invasive determination of glucose and or physiological parameters.

### Summary of the invention

According to the present invention, a simple, rapid and reliable determination of a physiological parameter is feasible using non-invasive systems and methods. These systems and methods involve irradiation of a body part of a subject, particular a human subject, with infrared visual (VIS)/rear-infrared (NIR) radiation in the range of about 500 nm to about 1500 nm and detecting emitted IR radiation from the irradiated body part of said subject in the range of about 5 µm to about 12 µm. Surprisingly, the present inventor has found that irradiating a body part, e.g. a fingertip, an earlobe, a wrist or a forearm with short-wavelength radiation and detecting emitted long-wavelength radiation from the irradiated body part allows determination for physiological parameters such as glucose in a bodily fluid such as blood

A first aspect of the invention relates to a non-invasive system for determining a physiological parameter, particularly glucose, in a bodily fluid of a subject comprising:
(a) a radiation source adapted for emitting visual (VIS)/near-infrared (NIR) radiation in the range of about 500 nm to about 1500 nm into a body part of said subject, wherein the body part is particularly selected from a fingertip, an earlobe, a wrist, and a forearm, and upper arm,
(b) a sensing unit for detecting emitted IR radiation from the irradiated body part of said subject in the range of about 5 µm to about 12 µm, wherein said sensing unit is adapted for (i) detecting IR radiation having at least one wavelength or wavelength range where the intensity of the detected IR radiation is dependent from the concentration of the physiological parameter in the bodily fluid of said subject, and for (ii) detecting IR radiation having at least one wavelength or wavelength range where the intensity of the detected IR radiation is substantially independent from the concentration of the physiological parameter in the bodily fluid of said subject, and
(c) an analyzing unit for the qualitative and/or quantitative determination of the physiological parameter based on the IR radiation detected in the sensing unit (b).

A further aspect of the invention relates to the use of the above system for non-invasively determining a physiological parameter in a bodily fluid of a subject, particularly wherein the physiological parameter is glucose, and the bodily fluid is blood.

A still further aspect of the invention relates to a method for non-invasively determining a physiological parameter, particularly glucose in a bodily fluid of a subject comprising the steps:
(a) irradiating a body part of said subject with visual (VIS)/near-infrared (NIR) radiation in the wavelength range of about 500 nm to about 1500 nm,
(b) detecting emitted IR radiation from the irradiated body part of said subject in the wavelength range of about 5 µm to about 12 µm, comprising separately (i) detecting IR radiation having at least one wavelength or wavelength range where the intensity of the detected IR radiation is dependent from the concentration of the physiological parameter in the bodily fluid of said subject, and (ii) detecting IR radiation having at least one wavelength or wavelength range where the intensity of the detected IR radiation is substantially independent from the concentration of the physiological parameter in the bodily fluid of said subject, and
(c) analyzing the detected IR radiation for the qualitative and/or quantitative determination of the physiological parameter.

### Detailed description

The present invention involves determination of a physiological parameter by detecting IR radiation from both previously irradiated body parts of a subject, particularly a human subject in the wavelength range of about 5 µm to about 12 µm, particularly in the range of about 8 µm to about 10 µm. The physiological parameter may be any compound having characteristic absorption bands in this wavelength range. For example, the physiological parameter is glucose or another clinically relevant analyte such as lactate or tropon in.

In a certain embodiment of the invention, the system is adapted for the non-invasive determination of glucose in blood. In this embodiment, IR radiation is detected at a glucose-specific wavelength or wavelength range where glucose has a characteristic absorption band and where the intensity of the detected IR radiation is dependent from the concentration of glucose in the blood. More particularly, the glucose-specific wavelength or wavelength range is selected from a wavelength of about 9.2 µm, a wavelength of about 9.4 µm, a wavelength of about 9.6 µm, a wavelength range comprising at least two of the wavelengths of about 9.2 µm, about 9.4 µm and about 9.6 µm, a wavelength range comprising all three of the wavelengths of about 9.2 µm, about 9.4 µm and about 9.6 µm or any combination thereof. Additionally, IR radiation is detected at a reference wavelength or wavelength range where glucose has no characteristic absorption band and particularly an absorption minimum and where the intensity of the detected IR radiation is substantially independent from the concentration of glucose in blood. More particularly, the reference wavelength or wavelength range is selected from a wavelength or wavelength range between about 8.7 µm to about 9.0 µm, a wavelength or a wavelength range between about 9.7 µm to about 10.2 µm or any combination thereof.

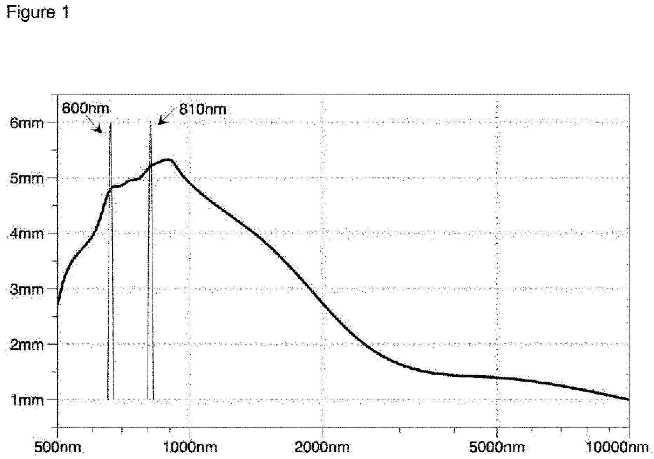

As outlined above, the invention is based on the irradiation of body tissue with electromagnetic radiation in the wavelength range between about 500 nm to about 1500 nm and detection of electromagnetic radiation emitted from the irradiated body part in the wavelength range between about 5 µm to about 15 µm. **Figure 1** shows the penetration depth of electromagnetic radiation into body tissue [mm] depending from the wavelength [nm]. It can be seen that the penetration depth is dependent from the wavelength. In the visual (VIS)/near-infrared (NIR) wavelength range between about 500 nm to about 1500 nm, particularly in the range of about 550 nm to about 1200 nm, there is a penetration depth of about 3 mm or more. Thus, a body part irradiated with radiation will absorb electromagnetic energy resulting in a local increase of tissue temperature. This again results in an increased emission of longer-wavelength IR radiation, e.g. IR radiation in the wavelength range of about 5 µm to about 12 µm, where certain organic compounds present in bodily fluids, i.e. physiological parameters, show absorption bands. This allows a quantitative or qualitative determination of such parameters according to the above aspects of the present invention.

In certain embodiments, the VIS/NIR radiation emitted into the body part is in the range of about 550 nm to about 1000 nm, particularly in the range of about 800 nm to about 820 nm, e.g. about 810 nm, and/or in the range of about 590 nm to about 660 nm, e.g. at about 600 nm.

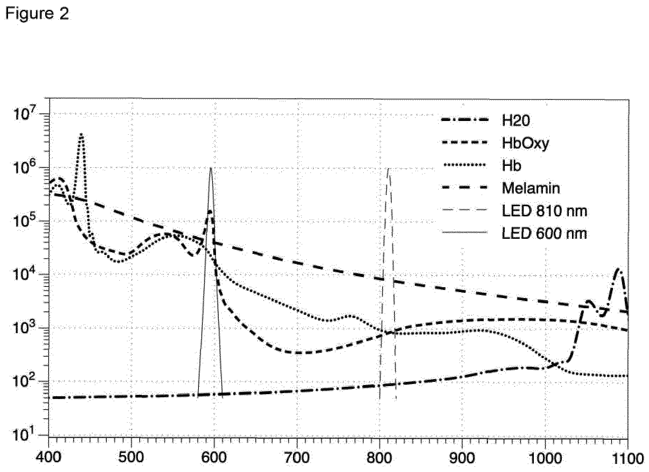

**Figure 2** shows relative absorption coefficients of certain compounds present in the human body depending from the wavelength in the range between 400 nm and 1100 nm. The wavelengths of 600 nm and 810 nm, at which radiation may be emitted into the body part, are specifically indicated. In the wavelength range of about 500 nm to about 1050 nm, there is a relatively low absorption of water (H₂O). Further, the major blood constituents hemoglobin (Hb) and oxyhemoglobin (Hboxy) show similar absorption coefficients. The skin pigment melamine shows an absorption coefficient which decreases with increasing wavelength.

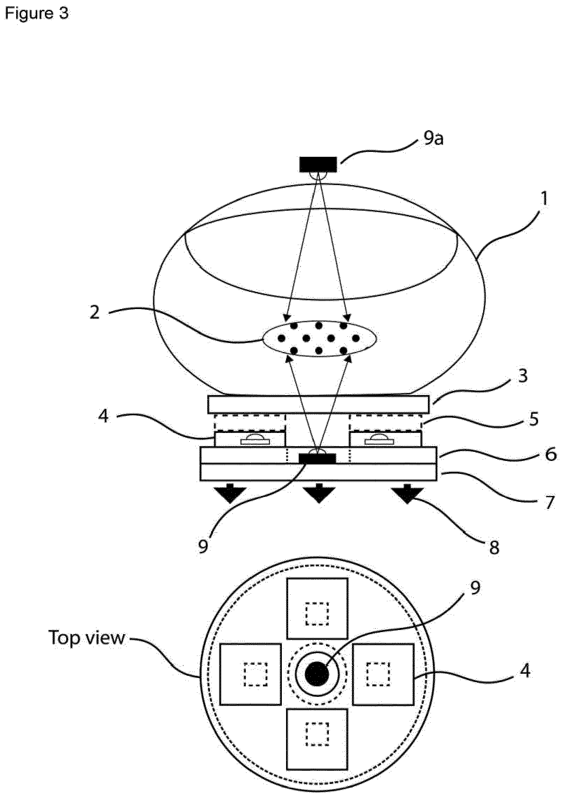

**Figure 3** shows an embodiment of a system of the present invention. A body part (1), e.g. a fingertip, is placed into contact with the system which is adapted to irradiate an absorption area (2) within the body part (1).

The system comprises a cover (3) which is at least partially made of a material which is optically transparent. For example, the cover is at least partially made of CaF₂ and/or BaF₂ and may have a thickness of about 0.2 mm to about 2 mm, particularly of about 0.5 mm to about 1.5 mm, more particularly about 1 mm.

The system further comprises at least one sensor (4) which may be provided with a filter element (5) and optionally a lens element (not shown) which e.g. may be arranged between a sensor (4) and a filter element (5). The sensor (4) may be mounted on a circuit board (6). Further, the system comprises at least one radiation source (9, 9a). For example, the system may comprise a radiation source (9) located on the same side as the sensor (4) and/or a radiation source (9a) located on an opposite side of the body part (1) with regard to the sensor (4). If desired, a further sensor (4) may be provided without filter element (5) for monitoring the exact skin temperature of the subject.

The system contains one or more sensors (4). In the embodiment of Figure 3, the system comprises four different sensors (4). The sensor may be an optical detector, particularly an optical photovoltaic detector, e.g. an InAsSb-based detector which may be used in combination with a lock-in amplifier, if desired. A photovoltaic detector, e.g. an InAsSb-based detector has a rise time of only few nanoseconds and is particularly useful in a set-up wherein the body part is irradiated intermittently. In other embodiments, the sensor may be heat detector, e.g. a thermopile or a bolometer. Suitable sensors include a photovoltaic detector (e.g. Hamamatsu P13894), a thermopile (e.g. Heimann HCS C21 F8-14) or other types of IR sensors (e.g. Sensirion STS21 or Melexis MLX90632). If desired, a sensor (4) may be provided with a filter element (5) capable of selectively transmitting radiation of a desired wavelength or wavelength range. The filter element may have narrow band width, e.g. of about 50 to 100 nm, or a broader band width, e.g. of about 400 nm or more. A filter may be made from germanium or other filter materials which are optically transmittant for the respective wavelengths. Further, a sensor may be provided with a lens element, e.g. a micro-lens capable of focusing the light falling onto the sensor.

In certain embodiments, the sensor is a miniaturized sensor having an area of about 1mm² to about 10,000 mm², e.g. of about 10mm² to about 1,000 mm². In certain embodiments, the sensor may be even more miniaturized, e.g. an ASIC (application-specific integrated circuit).

Further, the device may comprise a circuit board (7) on which the light source (9) is mounted and an active and/or passive heat sink (8).

The radiation source (9, 9a) may be adapted for emitting collimated radiation, e.g. a laser-based light source, and/or adapted for emitting non-collimated radiation, e.g. an LED-based light source. For example, the light source may be selected from an LED, a laser diode, a VCSEL (vertical-cavity surface-emitting laser) or a laser. The radiation source is adapted for emitting VIS/NIR radiation in the range of about 500 nm to about 1500 nm. The VIS/NIR radiation may be emitted continuously or intermittently throughout a predetermined time interval.

In a certain embodiment, the radiation source may be adapted for emitting radiation continuously at a power of about 10 mW to about 1 W, particularly for about 20 mW to about 500 mW, and more particularly of about 50 mW to about 250 mW for a time interval of about 0.1-20 s, particularly of about 1s to about 5s, and more particularly of about 0.5 s to about 2s.

In a further embodiment, the radiation source may be adapted for emitting radiation intermittently at a power of about 10 mW to about 5 W, particularly of about 20 mW to about 1 W, and more particularly of about 50 mW to about 500 mW for a time interval of about 0.1 s to about 20 s, particularly of about 0.2 s to about 5 s, and more particularly of about 0.5 s to about 2 s. The radiation may be emitted intermittently with a pulse frequency of about 1 Hz to about 1 MHz.

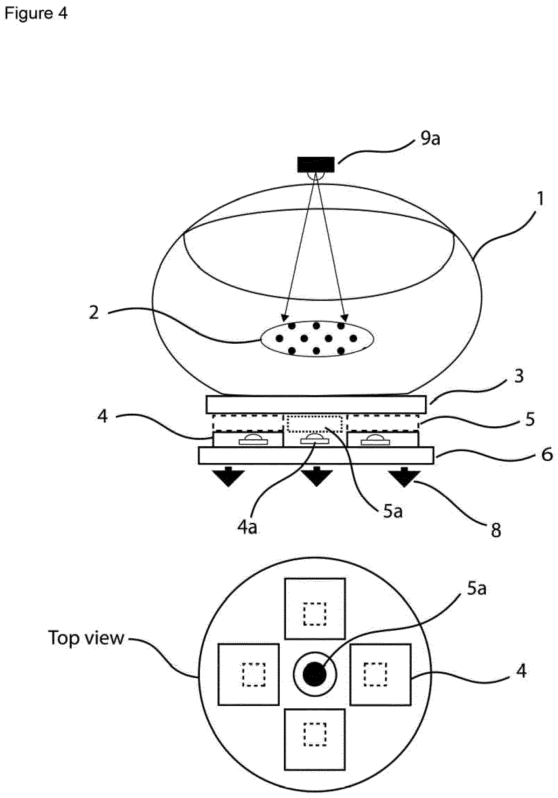

A further embodiment of the system of the invention is shown in **Figure 4****.** Here, a single radiation source (9a) is provided on a side of the body part (1) which is opposite to a sensing unit comprising at least one sensor (4) provided with a filter (5) and a further sensor (4a) provided with a filter (5a). In certain embodiments, sensor (4a) is an optical sensor, e.g. a photodiode. It is adapted for a reference measurement of transmission radiation from radiation source (9a), e.g. for measuring radiation at a wavelength of about 600 nm and/or 810 nm. For this purpose, filter element (5a) may be a bandpass filter at about 600 nm and/or 810 nm.

As shown in Figures 3 and 4, the system of the invention may comprise a plurality of different sensors (4). In certain embodiments, the system may comprise a plurality of analyte-specific, e.g. glucose-specific sensors wherein a first sensor is adapted for detecting radiation at a first wavelength or wavelength range, e.g. at a wavelength of about 9.2 µm and at least another first sensor is adapted for detecting IR radiation at a second wavelength range which encompasses the first wavelength or wavelength range and further comprises another wavelength or wavelength range. For example, the other first sensor may be adapted for detecting IR radiation at a wavelength of about 9.2 µm and additionally at a wavelength of about 9.4 µm and/or about 9.6 µm, particularly at a wavelength of about 9.4 µm and a wavelength of about 9.6 µm.

Further, the sensing unit may comprise a plurality of reference sensors adapted for detecting reference radiation at different wavelengths or wavelength ranges. For example, when determining glucose, a reference sensor may be adapted for detecting radiation having a wavelength range between about 8.6 µm and about 9.0 µm. Another reference sensor is adapted for detecting radiation at a wavelength or wavelength range between about 9.8 µm and about 10.2 µm.

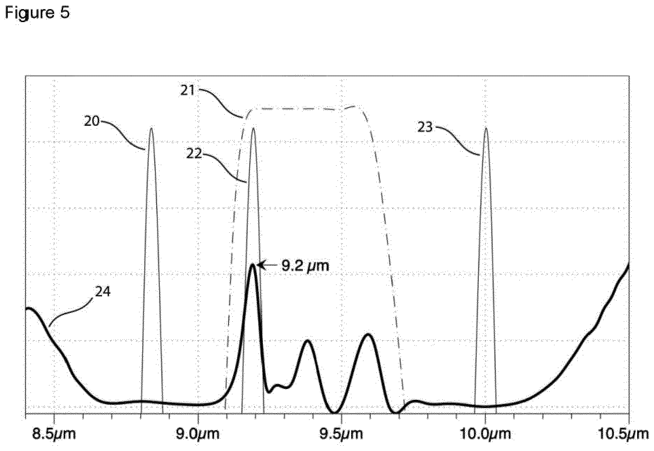

In **Figure 5****,** measurement at a plurality of analyte-specific wavelengths/wavelength ranges and reference wavelengths/wavelength ranges is shown.

The absorption signal of glucose (24) has three different peaks at about 9.2 µm, at about 9.4 µm and about 9.6 µm. A first glucose-specific sensor may be adapted for measuring only the peak at 9.2 µm. Such a sensor would be adapted with a filter element capable of transmitting radiation only in a narrow range (22). A further glucose-specific sensor may be adapted for measuring radiation at a broader range between about 9.1 µm and about 9.7 µm, thereby encompassing the peaks at about 9.2 µm, 9.4 µm and 9.6 µm. This sensor may be adapted with a filter element capable of transmitting radiation in a broader range (21).

Two reference sensors may be provided, capable of transmission of radiation with a wavelength in the range of about 8.8 µm - 8.9 µm (20) and/or radiation with a wavelength of about 9.9 µm - 10.1 µm (23), respectively.

Parallel and separate measurements at a wavelength of about 9.2 µm on the one hand and at a wavelength range including the peak at 9.2 µm, but also at least one of the other peaks, particularly the peak at about 9.6 µm have a further advantage, since they allow determination whether the subject's blood contains ethanol. Since ethanol and other alcohols have an absorption band at a wavelength of about 9.6 µm, but not at a wavelength of about 9.2 µm, the ratio between the peak at 9.2 µm and 9.6 µm may be used to determine and optionally correct a disturbance caused by blood alcohol.

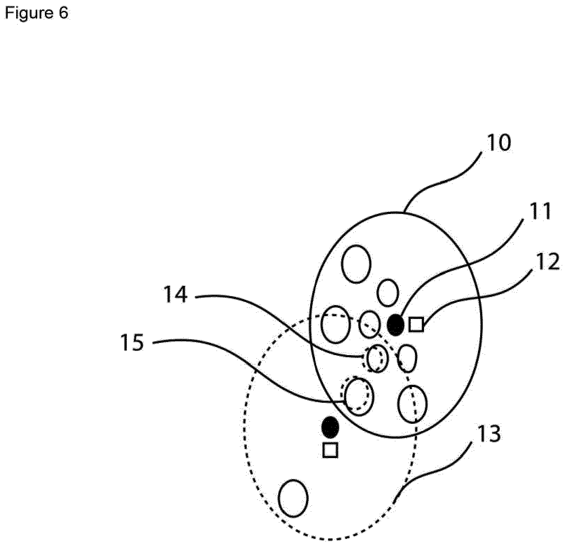

Still a further embodiment of the invention is shown in **Figure 6****.** Here, a system is provided comprising a filter wheel (10) capable of rotating around an axis (11) and a shutter wheel (13) also capable of rotating around an axis. The filter wheel and the shutter wheel are provided with illumination holes (15) through which light from the radiation source (not shown) may pass into the body part of the subject (not shown). Reflected light from the irradiated body part may pass through different holes (14) of the filter wheel which may be provided with analyte-specific filter elements and/or reference filter elements as described above. The filter wheel's (10) and the shutter wheel's (13) position may be monitored with a magnet (12) in combination with a magnetic sensor. In operation, they may be rotated with predetermined frequencies, thereby allowing passing of radiation from the radiation source into the body part and passing of radiation emitted from the body part at predetermined time intervals.

The system of the present invention further comprises an analyzing unit (c) for the qualitative and/or quantitative determination of a physiological parameter based on the IR radiation detected in the sensing unit (b). The analyzing unit may comprise, for example, an A/D converter and a microcontroller. The analysis of the measured signal may be based on the intensity and/or the decay time.

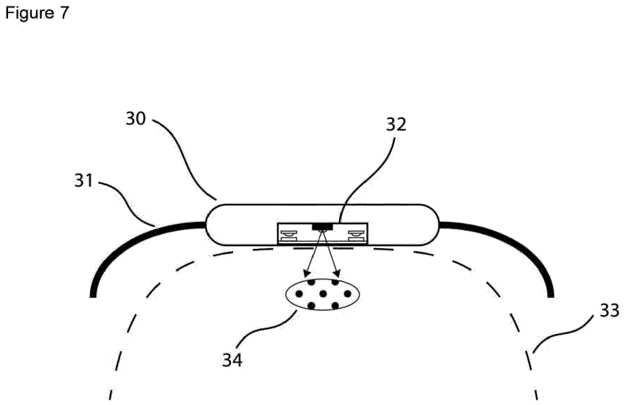

A still further embodiment of the invention is shown in **Figure 7****.** The system of this embodiment is adapted for being permanently fixed to the subject's body. This system is particularly adapted for carrying out a plurality of measurements in predetermined time intervals. The system comprises a housing (30) and a strap (31) for fixing the housing around the body (33), e.g. a wrist or forearm. Further, the system comprises a radiation source for emitting VIS/NIR light into an absorption area (34) of the body part (33) and sensors for detecting IR radiation emitted from the irradiated body part.

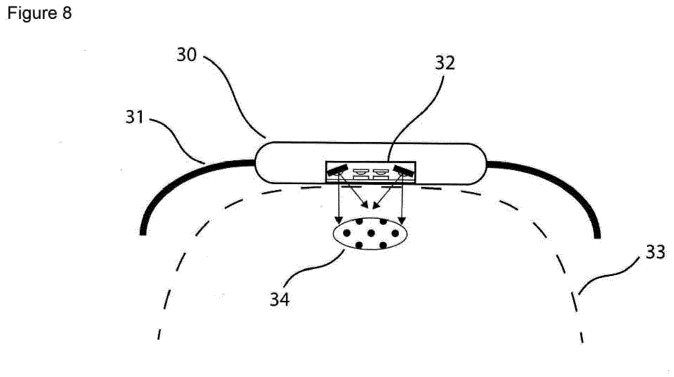

A still further embodiment of the invention is shown in **Figure 8****.** The system of this embodiment is adapted for being permanently fixed to the subject's body and particularly adapted for carrying out a plurality of measurements in predetermined time intervals. The system comprises a housing (30) and a strap (31) for fixing the housing around the body (33), e.g. a wrist or forearm. Further, the system comprises a plurality of radiation sources, e.g. 2 radiation sources, for emitting VIS/NIR light into an absorption area (34) of the body part (33) and sensors for detecting IR radiation emitted from the irradiated body part. The light emitted from these sources may fall at angle, e.g. at an angle of about 30° to about 75° onto the surface of the body part (33).

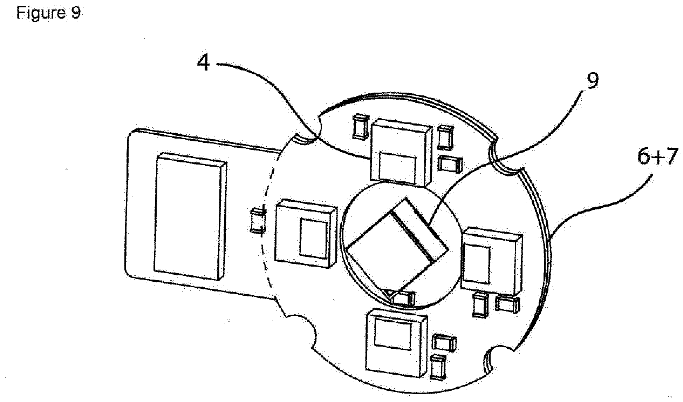

In **Figure 9****,** a schematic view of the system of Figure 3 is shown.

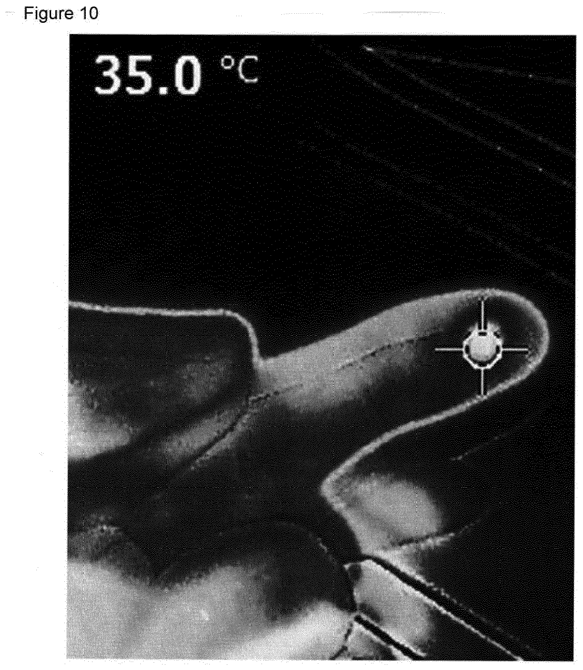

**Figure 10** shows a heat map of a fingertip after irradiation with light of 810 nm for a time period of 2s.

The system and the method of the invention allow qualitative and/or quantitative determination of the physiological parameter to be measured, particularly qualitative and/or quantitative determination of glucose in blood.

In certain embodiments, the concentration of the physiological parameter, e.g. the concentration of glucose in blood, is quantitatively determined. In certain embodiments, the alteration rate of the measured amount of the physiological parameter, e.g. glucose, is determined. These embodiments may involve a non-quantitative measurement, e.g. a relative measurement of the alteration of the analyte amount per time unit, i.e. the increase of the analyte amount or the decrease of the analyte amount per time unit. In case the alteration of the analyte amount into a single direction, i.e. increase or decrease, exceeds a predetermined level and/or time period, the system will provide an alert. This embodiment is particularly useful for systems as shown in Figure 7 and Figure 8, which may be permanently fixed at the body of the subject, e.g. around the wrist, forearm or upper arm.

A still further aspect of the invention is a non-invasive system for determining glucose in blood, which allows identification and optional correction of disturbances caused by blood alcohol comprising a sensing unit for detecting emitted IR radiation from a body part of said subject in the range of about 5 µm to about 12 µm,
wherein said sensing unit is adapted for (i) detecting IR radiation at a wavelength of about 9.2 µm and separately therefrom for detecting IR radiation at a wavelength of at least about 9.2 µm and about 9.6 µm, particularly at a wavelength range encompassing the wavelength of about 9.2 µm, about 9.4 µm and about 9.6 µm, and an analyzing unit for the separate determination of glucose from the above sensing units.

Still a further aspect of the invention is a method for non-invasively determining glucose in blood of a subject using this system.

Preferred features of these aspects are as previously indicated in the above specification.

Still a further aspect of the present invention is the use of an InAsSb sensor, optionally in combination with a lock-in amplifier for the measure of IR radiation emitted from a body part.

Preferred features of this aspect are as previously indicated in the above specification.

Still a further aspect of the present invention is a system and method for the non-quantitative measurement of glucose involving a plurality of measurements during a predetermined time interval and determining an alteration of the measurement signal indicating an alteration of the amount of analyte and providing an alter if the alteration of the glucose amount to one direction, i.e. increase or decrease, exceeds a certain level in a predetermined time period.

Preferred features of this aspect are as previously indicated in the above specification.

### Embodiments of the Specification

1. A non-invasive system for determining a physiological parameter in a bodily fluid of a subject comprising:
   (a) a radiation source adapted for emitting visual (VIS)/near-infrared (NIR) radiation in the range of about 500 nm to about 1500 nm into a body part of said subject,
   (b) a sensing unit for detecting emitted IR radiation from the irradiated body part of said subject in the range of about 5 µm to about 12 µm, wherein said sensing unit is adapted for (i) detecting IR radiation having at least one wavelength or wavelength range where the intensity of the detected IR radiation is dependent from the concentration of the physiological parameter in the bodily fluid of said subject,
      and for (ii) detecting IR radiation having at least one wavelength or wavelength range where the intensity of the detected IR radiation is substantially independent from the concentration of the physiological parameter in the bodily fluid of said subject, and
   (c) an analyzing unit for the qualitative and/or quantitative determination of the physiological parameter based on the IR radiation detected in the sensing unit (b).
2. The system of embodiment 1, wherein the physiological parameter is selected from compounds having at least one characteristic absorption band in the IR range of about 5 µm to about 12 µm, particularly in the range of about 8 µm to about 10 µm.
3. The system of embodiment 1 or 2, wherein the physiological parameter is glucose.
4. The system of any one of the preceding embodiments, wherein the bodily fluid is blood.
5. The system of any one of the preceding embodiments which is adapted for determining glucose in blood, wherein said sensing unit is adapted for detecting IR radiation having at least one wavelength or wavelength range where the intensity of the detected IR radiation is dependent from the concentration of glucose in the blood of said subject, wherein said at least one wavelength or wavelength range is particularly selected from a wavelength of about 9.2 µm, a wavelength of about 9.4 µm, a wavelength of about 9.6 µm, a wavelength range comprising at least two of the wavelengths of about 9.2 µm, about 9.4 µm and about 9.6 µm, a wavelength range comprising all three of the wavelengths of about 9.2 µm, about 9.4 µm and about 9.6 µm or any combination thereof.
6. The system of any one of the preceding embodiments which is adapted for determining glucose in blood, wherein said sensing unit is adapted for detecting IR radiation having at least one wavelength or wavelength range where the intensity of the detected IR radiation is substantially independent from the concentration of glucose in the blood of said subject, wherein said at least one wavelength or wavelength range is particularly selected from a wavelength or wavelength range between about 8.7 µm to about 9.0 µm, a wavelength or wavelength range between about 9.7 µm to about 10.2 µm or any combination thereof.
7. The system of any one of the preceding embodiments, wherein the radiation source (a) is adapted for emitting VIS/NIR radiation in the range of about 550 nm to about 1000 nm, particularly in the range of about 800 nm to about 820 nm, e.g. at about 810 nm, and/or in the range of about 590 nm to about 660 nm, e.g. at about 600 nm.
8. The system of any one of the preceding embodiments, wherein the radiation source (a) is adapted for emitting collimated radiation and/or adapted for emitting non-collimated radiation.
9. The system of any one of the preceding embodiments, wherein the radiation source (a) is a LED, a laser diode, a vcsel (vertical-cavity surface-emitting laser) or a laser.
10. The system of any one of the preceding embodiments, wherein the radiation source (a) is adapted for emitting VIS/NIR radiation continuously or intermittently throughout a predetermined time interval.
11. The system of embodiment 10, wherein the radiation source (a) is adapted for emitting VIS/NIR radiation continuously at a power of about 10 mW to about 1 W, particularly of about 20 mW to about 500 mW, and more particularly of about 50 mW to about 250 mW.
12. The system of embodiment 10 or 11, wherein the radiation source (a) is adapted for emitting VIS/NIR radiation continuously for a time interval of about 0.1 s to 20 s, particularly of about 1 s to about 5 s, and more particularly of about 0.5 s to about 2 s.
13. The system of embodiment 10, wherein the radiation source (a) is adapted for emitting VIS/NIR radiation intermittently at a power of about 10 mW to about 5 W, particularly of about 20 mW to about 1 W, and more particularly of about 50 mW to about 500 mW.
14. The system of embodiment 10 or 13, wherein the radiation source (a) is adapted for emitting VIS/NIR radiation intermittently for a time interval of about 0.1 s to about 20 s, particularly of about 0.2 s to about 5 s, and more particularly of about 0.5 s to about 2 s.
15. The system of embodiment 10, 13 or 14, wherein the radiation source (a) is adapted for emitting VIS/NIR radiation intermittently with a pulse frequency of about 1 Hz to about 1 MHz.
16. The system of any one of the preceding embodiments, wherein the sensing unit (b) comprises at least one first sensor, at least one second sensor, and optionally at least one third sensor,
   wherein the at least one first sensor is adapted for detecting IR radiation having at least one wavelength or wavelength range where the intensity of the detected IR radiation is dependent from the concentration of the physiological parameter in the bodily fluid of said subject,
   wherein the at least one second sensor is adapted for detecting IR radiation having at least one wavelength or wavelength range where the intensity of the detected IR radiation is substantially independent from the concentration of the physiological parameter in the bodily fluid of said subject, and
   wherein the at least one third sensor, if present, is adapted for detecting unspecific IR radiation.
17. The system of embodiment 16, wherein the at least one first sensor and the at least one second sensor are each provided with filter elements and optionally lens elements which are optically transparent in a predetermined wavelength or wavelength range.
18. The system of embodiment 16 or 17 comprising at least two different first sensors, which are adapted for detecting IR radiation having at least two different wavelengths or wavelength ranges.
19. The system of embodiment 16, 17 or 18, wherein at least one first sensor is adapted for detecting IR radiation having a first wavelength or wavelength range and at least one other first sensor is adapted for detecting IR radiation having a second wavelength range wherein the second wavelength range comprises the first wavelength or wavelength range and further comprises another wavelength or wavelength range.
20. The system of embodiment 19 for determining glucose in blood, wherein a first sensor is adapted for detecting IR radiation having a wavelength of about 9.2 µm and another first sensor is adapted for detecting IR radiation having a wavelength range between about 9.2 µm and about 9.6 µm which comprises the first wavelength of about 9.2 µm and further comprises at least one wavelength of about 9.4 µm and about 9.6 µm, and particularly comprises a wavelength of about 9.4 µm and a wavelength of about 9.6 µm.
21. The system of any one of embodiments 16-20 comprising at least two different second sensors, which are adapted for detecting IR radiation having at least two different wavelengths or wavelength ranges.
22. The system of embodiment 21 for determining glucose in blood, wherein a second sensor is adapted for detecting IR radiation having a wavelength or wavelength range between about 8.6 µm and 9.0 µm and another second sensor is adapted for detecting IR radiation having a wavelength or wavelength range between about 9.8 µm and about 10.2 µm.
23. The system of any of the preceding embodiments, wherein the sensing unit (b) comprises at least one sensor adapted for time-dependently and separately detecting IR radiation having different wavelengths or wavelength ranges,
   wherein in at least one first time interval the sensor is adapted for detecting IR radiation having at least one wavelength or wavelength range where the intensity of the detected IR radiation is dependent from the concentration of the physiological parameter in the bodily fluid of said subject, and
   wherein in at least one second time interval the sensor is adapted for detecting IR radiation having at least one wavelength or wavelength range where the intensity of the detected IR radiation is substantially independent from the concentration of the physiological parameter in the bodily fluid of said subject.
24. The system of embodiment 23, wherein the sensor is provided with a shutter wheel and/or a filter wheel.
25. The system of embodiment 24, wherein the shutter wheel comprises a plurality of openings wherein at least some of said openings are provided with filter elements and optionally lens elements which are optically transparent in a predetermined wavelength or wavelength range.
26. The system of any one of the preceding embodiments, wherein the sensing unit (b) comprises at least one sensor which is an optical detector, particularly an optical photovoltaics detector, more particularly an InAsSb-based detector.
27. The system of any one of the preceding embodiments, wherein the sensing unit (b) comprises at least one sensor which is a thermopile or a bolometer.
28. The system of any one of the preceding embodiments, wherein the analyzing unit (c) comprises a microcontroller adapted for quantitatively determining the concentration of the physiological parameter and/or for non-quantitatively determining the alteration rate of the physiological parameter.
29. The system of any one of the preceding embodiments, which is adapted for detecting IR radiation from a body part which is selected from a fingertip, an ear lobe, a wrist, a forearm and an upper arm.
30. The system of any one of the preceding embodiments, wherein the radiation source (a) and the sensing unit (b) are arranged on the same side of the body part.
31. The system of any one of the preceding embodiments, wherein the radiation source (a) and the sensing unit (b) are arranged on different sides, particularly on opposite sides of the body part.
32. The system of any one of the preceding embodiments, wherein a first radiation source (a) is arranged on the same side of the body part as the sensing unit (b) and a further radiation source (a) is arranged on a different side, particularly on an opposite side of the body part as the sensing unit.
33. The system of any one of the preceding embodiments further comprising a cover, wherein said cover is at least partially made of a material which is optically transparent for VIS/NIR radiation emitted by the radiation source (a) and for IR radiation detected by the sensing unit (b).
34. The system of embodiment 33, wherein the cover is at least partially made of CaF₂ and/or BaF₂.
35. The system of embodiment 33 or 34, wherein the optically transparent material of the cover has a thickness of about 0.2 mm to about 2 mm, particularly of about 0.5 mm to about 1.5 mm, more particularly about 1 mm.
36. Use of the system of any one of the preceding embodiments for non-invasively determining a physiological parameter in a bodily fluid of a subject.
37. The use of embodiment 36, wherein the physiological parameter is glucose and the bodily fluid is blood.
38. The use of embodiment 36 or 37, wherein the physiological parameter is quantitatively determined.
39. The use of embodiment 36, 37 or 38, wherein the alteration rate of the physiological parameter rate is non-quantitatively determined.
40. A method for non-invasively determining a physiological parameter in a bodily fluid of a subject comprising the steps:
   (a) irradiating a body part of said subject with visual (VIS)/near-infrared (NIR) radiation in the wavelength range of about 500 nm to about 1500 nm,
   (b) detecting emitted IR radiation from the irradiated body part of said subject in the wavelength range of about 5 µm to about 12 µm, comprising separately
      (i) detecting IR radiation having at least one wavelength or wavelength range where the intensity of the detected IR radiation is dependent from the concentration of the physiological parameter in the bodily fluid of said subject, and
      (ii) detecting IR radiation having at least one wavelength or wavelength range where the intensity of the detected IR radiation is substantially independent from the concentration of the physiological parameter in the bodily fluid of said subject, and
   (c) analyzing the detected IR radiation for the qualitative and/or quantitative determination of the physiological parameter.
41. The method of embodiment 40, wherein the physiological parameter is glucose and the bodily fluid is blood.
42. The method of embodiment 40 or 41, wherein the physiological parameter is quantitatively determined.
43. The method of embodiment 40, 41 or 42, wherein the alteration rate of the physiological parameter rate is non-quantitatively determined.

## Claims

1. A non-invasive system for determining a physiological parameter in a bodily fluid of a subject comprising:
(a) a radiation source adapted for emitting visual (VIS)/near-infrared (NIR) radiation in the range of about 500 nm to about 1500 nm into a body part of said subject, wherein the body part is particularly selected from a fingertip, an ear lobe, a wrist, a forearm, and upper arm,
(b) a sensing unit for detecting emitted IR radiation from the irradiated body part of said subject in the range of about 5 µm to about 12 µm, wherein said sensing unit is adapted for (i) detecting IR radiation having at least one wavelength or wavelength range where the intensity of the detected IR radiation is dependent from the concentration of the physiological parameter in the bodily fluid of said subject,
and for (ii) detecting IR radiation having at least one wavelength or wavelength range where the intensity of the detected IR radiation is substantially independent from the concentration of the physiological parameter in the bodily fluid of said subject, and
(c) an analyzing unit for the qualitative and/or quantitative determination of the physiological parameter based on the IR radiation detected in the sensing unit (b).

2. The system of claim 1, wherein the physiological parameter is glucose and the bodily fluid is glucose.

3. The system of claim 1 or 2 which is adapted for determining glucose in blood, wherein said sensing unit is adapted for detecting IR radiation having at least one wavelength or wavelength range where the intensity of the detected IR radiation is dependent from the concentration of glucose in the blood of said subject, wherein said at least one wavelength or wavelength range is particularly selected from a wavelength of about 9.2 µm, a wavelength of about 9.4 µm, a wavelength of about 9.6 µm, a wavelength range comprising at least two of the wavelengths of about 9.2 µm, about 9.4 µm and about 9.6 µm, a wavelength range comprising the wavelengths of about 9.2 µm, about 9.4 µm and about 9.6 µm or any combination thereof.

4. The system of any one of the preceding claims, wherein the radiation source (a) is adapted for emitting VIS/NIR radiation in the range of about 550 nm to about 1200 nm, particularly in the range of about 800 nm to about 820 nm, e.g. at about 810 nm, and/or in the range of about 590 nm to about 610 nm, e.g. at about 600 nm.

5. The system of any one of the preceding claims, wherein the radiation source (a) is a LED, a laser diode, a vcsel (vertical-cavity surface-emitting laser) or a laser.

6. The system of claim any one of the preceding claims, wherein the sensing unit (b) comprises at least one first sensor, at least one second sensor, and optionally at least one third sensor,
wherein the at least one first sensor is adapted for detecting IR radiation having at least one wavelength or wavelength range where the intensity of the detected IR radiation is dependent from the concentration of the physiological parameter in the bodily fluid of said subject,
wherein the at least one second sensor is adapted for detecting IR radiation having at least one wavelength or wavelength range where the intensity of the detected IR radiation is substantially independent from the concentration of the physiological parameter in the bodily fluid of said subject, and
wherein the at least one third sensor, if present, is adapted for detecting unspecific IR radiation.

7. The system of claim 6, wherein at least one first sensor is adapted for detecting IR radiation having a first wavelength or wavelength range and at least one other first sensor is adapted for detecting IR radiation having a second wavelength range,
wherein the second wavelength range comprises the first wavelength or wavelength range and further comprises another wavelength or wavelength range,
wherein the system is particularly adapted for determining glucose in blood, wherein a first sensor is adapted for detecting IR radiation having a wavelength of about 9.2 µm and another first sensor is adapted for detecting IR radiation having a wavelength range between about 9.2 µm and about 9.6 µm which comprises the first wavelength of about 9.2 µm and further comprises at least one wavelength of about 9.4 µm and about 9.6 µm, and particularly further comprises a wavelength of about 9.4 µm and about 9.6 µm.

8. The system of claim 6 or 7 comprising at least two different second sensors, which are adapted for detecting IR radiation having at least two different wavelengths or wavelength ranges,
wherein the system is particularly adapted for determining glucose in blood, wherein a second sensor is adapted for detecting IR radiation having a wavelength or wavelength range between about 8.6 µm and 9.0 µm and another second sensor is adapted for detecting IR radiation having a wavelength or wavelength range between about 9.8 µm and about 10.2 µm.

9. The system of any of the preceding claims, wherein the sensing unit (b) comprises at least one sensor adapted for time-dependently and separately detecting IR radiation having different wavelengths or wavelength ranges,
wherein in at least one first time interval the sensor is adapted for detecting IR radiation having at least one wavelength or wavelength range where the intensity of the detected IR radiation is dependent from the concentration of the physiological parameter in the bodily fluid of said subject, and
wherein in at least one second time interval the sensor is adapted for detecting IR radiation having at least one wavelength or wavelength range where the intensity of the detected IR radiation is substantially independent from the concentration of the physiological parameter in the bodily fluid of said subject.

10. The system of any one of the preceding claims, wherein the sensing unit (b) comprises at least one sensor which is an optical detector, particularly an optical photovoltaics detector, more particularly an InAsSb-based detector.

11. The system of any one of the preceding claims further comprising a cover, wherein said cover is at least partially made of a material which is optically transparent for IR/VIS radiation emitted by the radiation source (a) and for IR radiation detected by the sensing unit (b), wherein the cover is at least partially made of CaF₂ and/or BaF₂ and wherein the optically transparent material of the cover has a thickness of about 0.2 mm to about 2 mm, particularly of about 0.5 mm to about 1.5 mm, more particularly about 1 mm.

12. Use of the system of any one of the preceding claims for non-invasively determining a physiological parameter in a bodily fluid of a subject, wherein the physiological parameter is glucose and the bodily fluid is blood, and wherein the alteration rate of the amount of glucose in blood is determined.

13. A method for non-invasively determining a physiological parameter in a bodily fluid of a subject comprising the steps:
(a) irradiating a body part of said subject with visual (VIS)/near-infrared (NIR) radiation in the wavelength range of about 500 nm to about 1500 nm,
(b) detecting emitted IR radiation from the irradiated body part of said subject in the wavelength range of about 5 µm to about 12 µm, comprising separately
(i) detecting IR radiation having at least one wavelength or wavelength range where the intensity of the detected IR radiation is dependent from the concentration of the physiological parameter in the bodily fluid of said subject, and
(ii) detecting IR radiation having at least one wavelength or wavelength range where the intensity of the detected IR radiation is substantially independent from the concentration of the physiological parameter in the bodily fluid of said subject, and
(c) analyzing the detected IR radiation for the qualitative and/or quantitative determination of the physiological parameter.

14. The method of claim 13, wherein the physiological parameter is glucose and the bodily fluid is blood.

15. The method of claim 13 or 14, wherein the concentration of the physiological parameter is quantitatively determined, and/or wherein the alteration rate of the amount of the physiological parameter is determined.
